# EUROPEAN PATENT SPECIFICATION

(11) **EP 3 485 803 B1**
(45) Date of publication and mention of the grant of the patent: **04.11.2020**
(21) Application number: 18183026.6
(22) Date of filing: 11.07.2018
(51) Int. Cl.: A61B 5/00, A61B 5/0205, A61B 5/0402, A61B 5/11

(54) **WEARABLE DEVICE CAPABLE OF RECOGNIZING SLEEP STAGE AND RECOGNITION METHOD THEREOF**
WEARABLE-VORRICHTUNG ZUR DETEKTION DES SCHLAFSTADIUMS UND DETEKTIONSVERFAHREN DAFÜR
DISPOSITIF PORTABLE POUVANT RECONNAÎTRE UN STADE DE SOMMEIL ET SON PROCÉDÉ DE RECONNAISSANCE

(30) Priority: 20.11.2017 TW 106140053
(43) Date of publication of application: 22.05.2019
(73) Proprietor: Kinpo Electronics, Inc., New Taipei City 22201 (TW)
(72) Inventor: HARAIKAWA, Koichi, 22201 New Taipei City (TW); CHIEN, Jen-Chien, 22201 New Taipei City (TW); LIN, Yin-Tsong, 22201 New Taipei City (TW); HUNG, Tsui-Shan, 22201 New Taipei City (TW); LIN, Chien-Hung, 22201 New Taipei City (TW)
(74) Representative: Emde, Eric

(56) References cited:
- WO-A1-2016/135382
- WO-A1-2017/079828
- US-A1- 2017 215 808
- Ricardo Marques Trindade: "Detection of sleep position by means of a wrist-worn sensor", , 1 November 2016 (2016-11-01), pages 1-10, XP055529675, Retrieved from the Internet: URL:https://fenix.tecnico.ulisboa.pt/downl oadFile/1689244997256787/ExtendedAbstract. pdf [retrieved on 2018-12-04]

## Description

### Technical Field

The disclosure relates to a wearable device, and more particularly, to a wearable device capable of recognizing sleep stage and a recognition method thereof.

### BACKGROUND

In the technical field for recognizing sleep stage, the common sleep stage recognition is usually conducted by assessment with a polysomnography (PSG) system through multiple physiological sensing operations. However, the physiological sensing operations include, for example, electroencephalography (EEG), electrooculography (EOG), electrocardiogram (ECG), electromyography (EMG), respiratory Effort, air flow, blood pressure, blood oxygen saturation (SaO₂), and heart rate and sleep gesture. In other words, the common sleep stage recognition requires complicated accessories to be worn by a wearer and requires analysis on a large amount of sense data. In consideration of the above, providing a wearable device capable of effectively sensing a sleep stage of the wearer having characteristics of convenience is one of important issues to be addressed in the field.

US 2017/215808 A1 relates to systems and methods for probabilistically estimating an individual's sleep stage based on spectral analyses of pulse rate and motion data. In one implementation, the method may include receiving signals from sensors worn by the individual, the signals including a photoplethysmographic (PPG) signal and an accelerometer signal; dividing the PPG signal into segments; determining a beat interval associated with each segment; resampling the set of beat intervals to generate an interval signal; and generating signal features based on the interval signal and the accelerometer signal, including a spectrogram of the interval signal. The method may further include determining a sleep stage for the individual by comparing the signal features to a sleep stage classifier included in a learning library. The sleep stage classifier may include one or more functions defining a likelihood that the individual is in the sleep stage based on the signal features.

WO 2017/079828 A1 discloses an apparatus for measuring cardiopulmonary data of a wearer, comprising: a sensor operable to produce a data stream indicative of movements of a wearer's body; a positioning device holding said sensor proximate an anatomical landmark on said wearer's body for conduction of mechanical vibrations from said wearer's body to said sensor; and a processor configured to receive said data stream and produce a rate signal indicative of cardiac or respiratory rate data of said wearer using an algorithm comprising peak detection.

WO 2016/135382 A1 discloses a method for assessing readiness of a user, said method comprising obtaining the user's movements; using the obtained user's movements to determine a nature of the period, wherein the nature of the period is selected from an activity period and a rest period; measuring at least one biosignal of the user during the rest period; determining a rest summary for the rest period, based on the measured at least one biosignal and at least one biosignal of a previous rest period; determining an activity summary for the activity period, based on the obtained movements of an activity period and obtained movements of at least one previous activity period; determining a body response summary based on the rest summary and the activity summary; and calculating a readiness score based on the body response summary and a previous body response summary, whereby the readiness score indicates a level of readiness of the user. The method is implemented in a system using a ring worn by the user.

Ricardo Marques Trindade, "Detection of sleep position by means of a wrist-worn sensor", 1 November 2016, pages 1 - 10, URL: https:// fenix.tecnico. ulisboa.pt/downloadFile/1689244997256787/ExtendedAbstract.pdf, discloses an answer to the question: Is it possible to develop an algorithm that classifies the sleeping position, solely based on a three-axis accelerometer embedded in a smartwatch? For this, a sleep movement database was built. To acquire sleep movements an Android application was developed that allowed to record accelerometer readings from a smartwatch. A wrist accelerometer was worn by patients from Hospital de Santa Maria, and volunteers while they slept. The data obtained from these nights was used to assess the algorithm's performance. The developed algorithm uses classifiers such as Neural Networks, Support Vector Machines, and the Naïve Bayes classifier. The core task is to identify the sleep movement performed and from there infer the position. When defining only 3 possible sleep positions (supine, lateral and prone) the average algorithm accuracy was 53.2±26.9% for neural networks, 51±20.9% for support vector machines, 44.5±15.9% for the Naive Bayes classifier and 35.6±21.1% with Hidden Markov models. This may have paved the way for the development of an algorithm that classifies sleep position using only an accelerometer placed on the wrist, and it is suggested using recurrent neural networks and trying other devices. The algorithm's core task is to classify which movement was performed and from there infer the position.

### SUMMARY

The disclosure provides a wearable device capable of recognizing sleep stage and a method thereof, which can effectively recognize the sleep stage of the wearer and provide characteristics of convenience.

According to an aspect of the present invention, a wearable device capable of recognizing sleep stage is provided as set forth in claim 1. According to another aspect of the present invention, a recognition method of sleep stage is provided as set forth in claim 9. Preferred embodiments of the present invention may be gathered from the dependent claims.

Based on the above, the wearable device capable of recognizing sleep stage and the method thereof according to the disclosure can sense a plurality of characteristic values by the electrocardiogram sensor, the acceleration sensor and the angular acceleration sensor. In this way, the processor can utilize the trained neural network module to perform the sleep stage recognition operation according to the characteristic values. As a result, the wearable device of the disclosure can effectively obtain a recognition result of the sleep stage and provide characteristics of convenience.

To make the above features and advantages of the disclosure more comprehensible, several embodiments accompanied with drawings are described in detail as follows.

### BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.
FIG. 1 illustrates a block diagram of a wearable device in an embodiment of the disclosure.
FIG. 2 illustrates a waveform diagram of an electrocardiogram signal in an embodiment of the disclosure.
FIG. 3 illustrates a block diagram for analyzing the electrocardiogram signal in an embodiment of the disclosure.
FIG. 4 illustrates a waveform diagram of a plurality of first characteristic values in an embodiment of the disclosure.
FIG. 5 illustrates a waveform diagram of another plurality of first characteristic values in an embodiment of the disclosure.
FIG. 6 illustrates a block diagram for analyzing an acceleration signal in an embodiment of the disclosure.
FIG. 7 illustrates a waveform diagram of a plurality of second characteristic values in an embodiment of the disclosure.
FIG. 8 illustrates a block diagram for analyzing an angular acceleration signal in an embodiment of the disclosure.
FIG. 9 illustrates a flowchart of a recognition method of sleep stage in an embodiment of the disclosure.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

In order to make content of the disclosure more comprehensible, embodiments are provided below to describe the disclosure in detail, however, the disclosure is not limited to the provided embodiments, and the provided embodiments can be suitably combined. Moreover, elements/components/steps with same reference numerals represent same or similar parts in the drawings and embodiments.

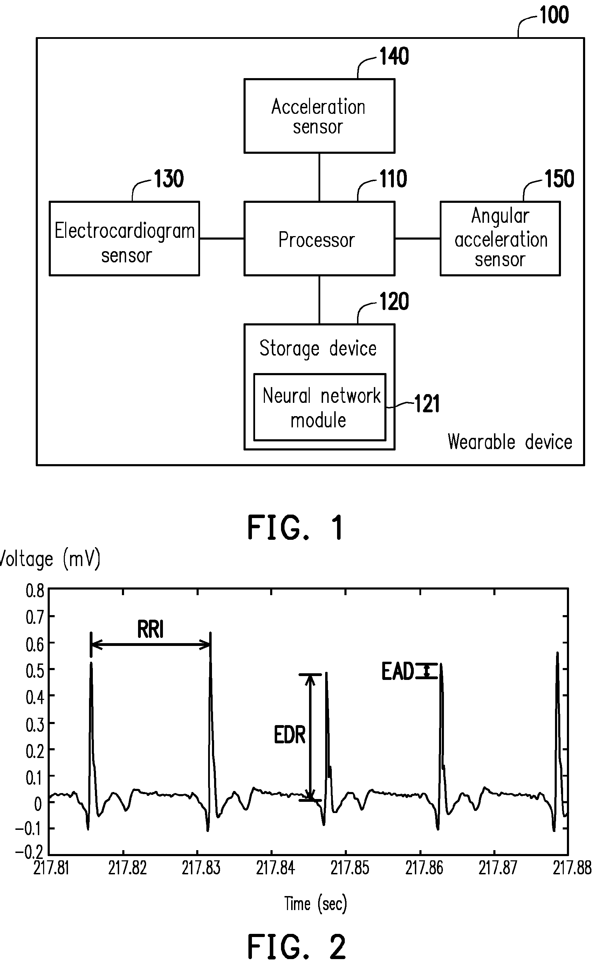

FIG. 1 illustrates a block diagram of a wearable device in an embodiment of the disclosure. With reference to FIG. 1, a wearable device 100 includes a processor 110, a storage device 120, an electrocardiogram sensor 130, an acceleration sensor 140 and an angular acceleration sensor 150. The processor 110 is coupled to the storage device 120, the electrocardiogram sensor 130, the acceleration sensor 140 and the angular acceleration sensor 150. In the present embodiment, the wearable device 100 may be, for example, smart clothes, smart wristbands or other similar devices, and the wearable device 100 is configured to recognize the sleep stage of the wearer. The wearable device 100 can integrate each of the sensors into one wearable item instead of complicated accessories. In other words, when the wearer is asleep, the sleep stage of the wearer may be sensed by the wearable device 100 worn by the wearer. In the present embodiment, a sleep recognition may be conducted for the wearer in a lying or sitting posture, for example, so as to monitor a sleep quality of the wearer and record a sleep situation. Further, in the present embodiment, the sleep stage recognizable by the wearable device 100 can include a wakefulness stage (Stage W), a first non-rapid eye movement stage (Stage N1, a.k.a. falling-asleep stage), a second non-rapid eye movement stage (Stage N2, a.k.a. slightly-deeper sleep stage), a third non-rapid eye movement stage (Stage N3, a.k.a. deep sleep stage) and a rapid eye movement (Stage R) according to Polysomnography (PSG). It should be noted that, a usage scenario of the wearable device 100 of the disclosure refers to a usage scenario in which the wearer is in the sleep situation where the deep sleep stage may take place in a longer period of time.

In the present embodiment, the electrocardiogram sensor 130 is configured to sense an electrocardiogram signal ECG and provide the electrocardiogram signal to the processor 110. The processor 110 analyzes the electrocardiogram signal to generate a plurality of first characteristic values. In the present embodiment, the acceleration sensor 140 is configured to sense an acceleration caused by moving of the wearer's body or wearing part and provide an acceleration signal to the processor 110. The processor 110 analyzes the acceleration signal to generate a plurality of second characteristic values. In the present embodiment, the angular acceleration sensor 150 is configured to sense an angular acceleration caused by turning of the wearer's body or wearing part and provide an angular acceleration signal to the processor 110. The processor 110 analyzes the angular acceleration signal to generate a plurality of third characteristic values. In other words, when the wearer is asleep, because the wearer may intentionally or unintentionally turn over or move his/her body, the wearable device 100 of the present embodiment can also sense the effect of turning or moving of the wearing part of the wearer in addition to sensing the electrocardiogram information of the wearer, such an analysis result provided by the processor 110 can take the effect caused by actions of the wearer in consideration.

In the present embodiment, the processor 110 is, for example, a central processing unit (CPU), a system on chi (SOC) or other programmable devices for general purpose or special purpose, such as a microprocessor and a digital signal processor (DSP), a programmable controller, an application specific integrated circuit (ASIC), a programmable logic device (PLD) or other similar devices or a combination of above-mentioned devices.

In the present embodiment, the storage device 120 is, for example, a dynamic random access memory (DRAM), a flash memory or a non-volatile random access memory (NVRAM). In the present embodiment, the storage device 120 is configured to store data and program modules described in each embodiment of the disclosure, which can be read and executed by the processor 110 so the wearable device 100 can realize the recognition method of sleep stage described in each embodiment of the disclosure.

In the present embodiment, the storage device 120 further includes a neural network module 121. The processor 110 can sense a plurality of characteristic values from different wearers in advance by the electrocardiogram sensor 130, the acceleration sensor 140 and the angular acceleration sensor 150, and use the characteristic values from the different wearers as sample data. In the present embodiment, the processor 110 can create a prediction model according to determination conditions, algorithms and parameters from the sleep stage, and use a plurality of the sample data for training or correcting the prediction model. Accordingly, when a sleep stage recognition operation is performed by the wearable device 100 for the wearer, the processor 110 can utilize the trained neural network module 121 to obtain a recognition result according to the first characteristic values, the second characteristic values and the third characteristic values sensed by the electrocardiogram sensor 130, the acceleration sensor 140 and the angular acceleration sensor 150. Nevertheless, enough teaching, suggestion, and implementation illustration regarding algorithms and calculation modes for the trained neural network module 121 of the present embodiment may be obtained with reference to common knowledge in the related art, which is not repeated hereinafter.

FIG. 2 illustrates a waveform diagram of an electrocardiogram signal in an embodiment of the disclosure. With reference to FIG. 1 and FIG. 2, the electrocardiogram signal sensed by the electrocardiogram sensor 130 is as shown in FIG. 2. In the present embodiment, the processor 110 can perform a heart rate variability (HRV) analysis operation to analyze a heart beat interval variation (R-R interval) RRI of the electrocardiogram signal, so as to obtain a plurality R-wave signals in the electrocardiogram signal. The processor 110 can perform the heart rate variability analysis operation to analyze variation of the R-wave signals. Further, the processor 110 can also perform a R-wave amplitude analysis operation and an adjacent R-waves difference (Difference of Amplitude between R and next R, EAD) analysis operation to analyze the R-wave signals. For instance, in the present embodiment, a distance between two R-waves may be used as the heart beat interval variation RRI. The R-wave amplitude analysis operation is, for example, to analyze a R-wave amplitude EDR in the electrocardiogram in order to obtain an ECG-derived respiratory signal, wherein peak and trough of the R-wave may be used as the R-wave amplitude EDR. A difference between the peaks of two adjacent R-waves may be used as the adjacent R-waves difference.

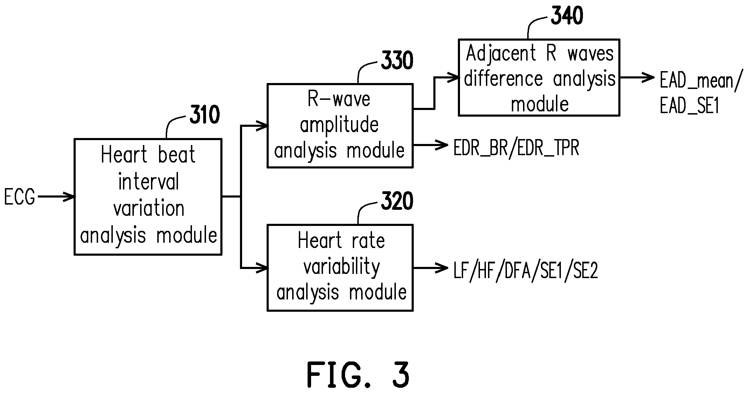

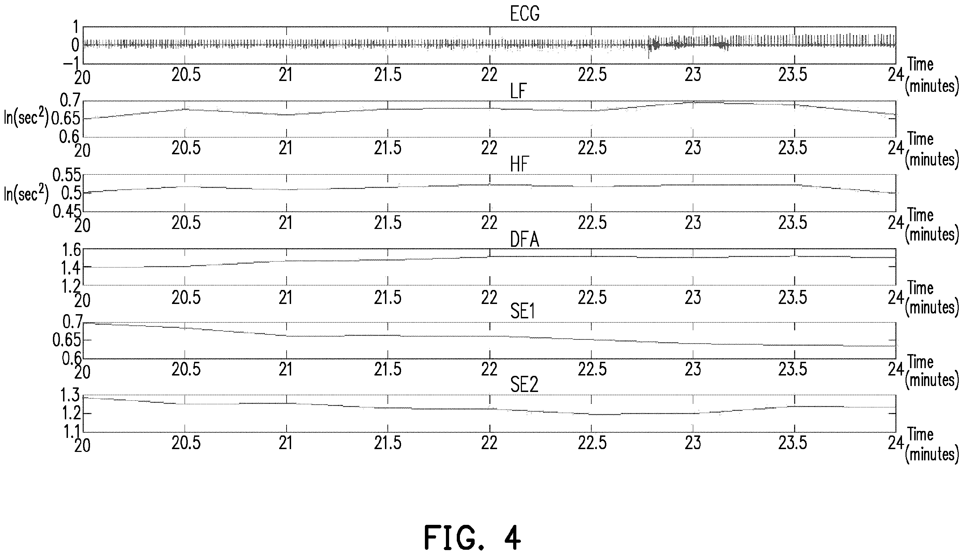

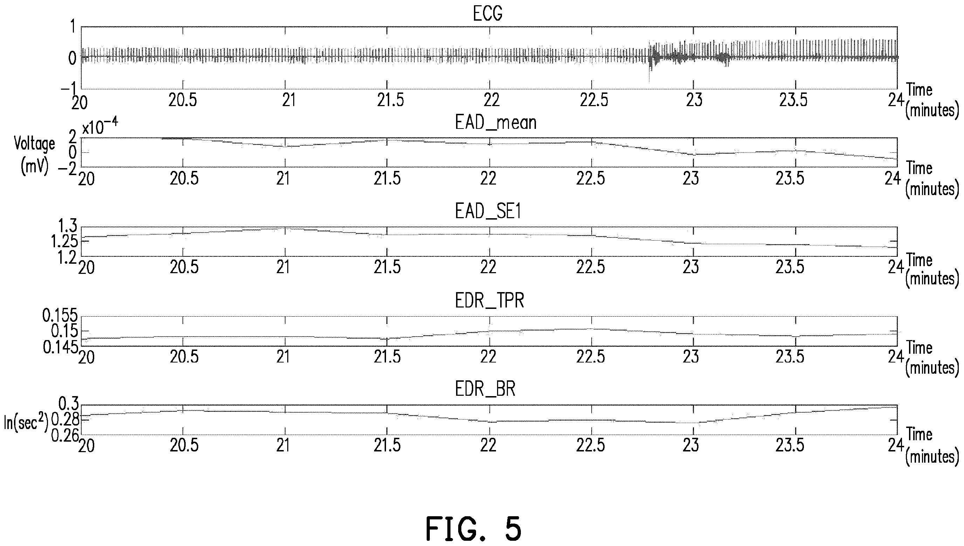

FIG. 3 illustrates a block diagram for analyzing the electrocardiogram signal in an embodiment of the disclosure. FIG. 4 illustrates a waveform diagram of a plurality of first characteristic values in an embodiment of the disclosure. FIG. 5 illustrates a waveform diagram of another plurality of first characteristic values in an embodiment of the disclosure. It should be noted that, each of the following waveform diagrams shows, for example, a sleep recognition operation performed per 30 seconds within a time length of 5 minutes. With reference to FIG. 1 to FIG. 5, the storage device 120 can store, for example, a heart beat interval variation analysis module 310, a heart rate variability analysis module 320, a R-wave amplitude analysis module 330 and an adjacent R-waves difference analysis module 340. In the present embodiment, the processor 110 receives the electrocardiogram signal ECG of the wearer provided by the electrocardiogram sensor, and analyzes the electrocardiogram signal ECG by the heart beat interval variation analysis module 310, so as to obtain a plurality of R-wave signal as shown in FIG. 2.

In the present embodiment, the heart rate variability analysis module 320 analyzes the R-wave signals, so as to obtain a low frequency signal LF, a high frequency signal HF, a detrended fluctuation analysis signal DFA, a first sample entropy signal SE1 and a second sample entropy signal SE2 as shown in FIG. 4. In the present embodiment, the low frequency signal LF is, for example, a signal with strength ranged from 0.04Hz to 0.15Hz among the R-wave signals. The high frequency signal HF is, for example, a signal with strength ranged from 0.15Hz to 0.4Hz among the R-wave signals. The detrended fluctuation analysis signal DFA is, for example, a signal underwent a detrended fluctuation analysis (DFA) among the R-wave signals. The first sample entropy signal SE1 is, for example, a signal underwent a sample entropy operation with the number of samples being 1 among the R-wave signals. The second sample entropy signal SE2 is, for example, a signal underwent a sample entropy operation with the number of samples being 2 among the R-wave signals.

In the present embodiment, the R-wave amplitude analysis module 330 analyzes the R-wave signals, so as to obtain a result including a turning point ratio value EDR_TPR and a signal strength value EDR_BR as shown in FIG. 5. Further, in the present embodiment, the adjacent R-waves difference analysis module 340 analyzes the R-wave signals, so as to obtain a mean value EAD_mean and a sample entropy value EAD_SE1 as shown in FIG. 5. In other words, the first characteristic values of the present embodiment are obtained from the low frequency signal LF, the high frequency signal HF, the detrended fluctuation analysis signal DFA, the first sample entropy signal SE1 and the second sample entropy signal SE2, and include the turning point ratio value EDR_TPR, the signal strength value EDR_BR, mean value EAD_mean and a sample entropy value EAD_SE1.

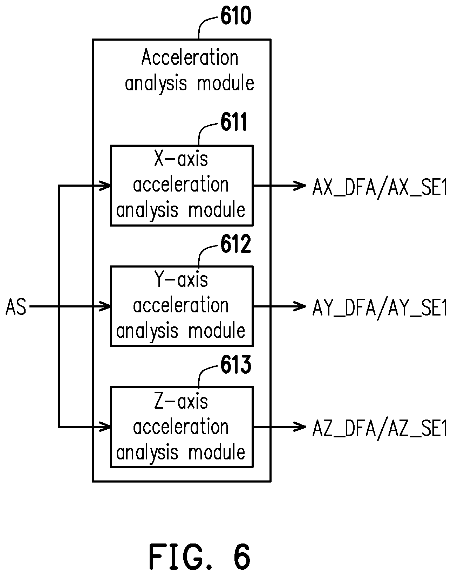

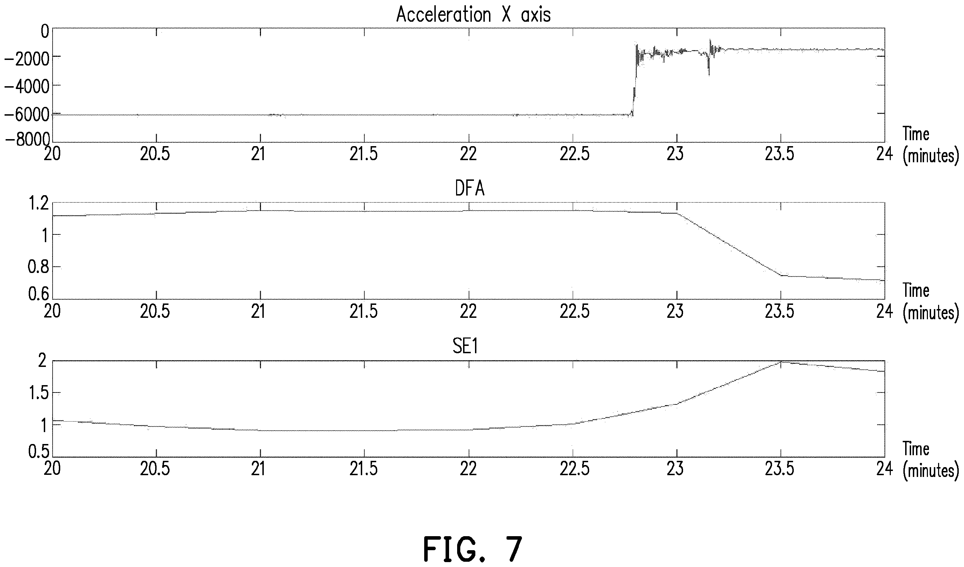

FIG. 6 illustrates a block diagram for analyzing an acceleration signal in an embodiment of the disclosure. FIG. 7 illustrates a waveform diagram of a plurality of second characteristic values in an embodiment of the disclosure. With reference to FIG. 1, FIG. 6 and FIG. 7, the storage device 120 can store, for example, an acceleration analysis module 610. In the present embodiment, the acceleration analysis module 610 includes an X-axis acceleration analysis module 611, a Y-axis acceleration analysis module 612 and a Z-axis acceleration analysis module 613. The acceleration sensor 140 can sense an acceleration signal AS of the wearer, wherein the acceleration sensor 140 may be, for example, a gravity sensor (G sensor). The acceleration analysis module 610 can analyze the acceleration signal AS to obtain respective acceleration values of X-axis (a first direction axis), Y-axis (a second direction axis) and Z-axis (a third direction axis), and obtain respective acceleration detrended fluctuation analysis values and respective acceleration sample entropy values by the X-axis acceleration analysis module 611, the Y-axis acceleration analysis module 612 and the Z-axis acceleration analysis module 613. For instance, the X-axis acceleration analysis module 611, the Y-axis acceleration analysis module 612 and the Z-axis acceleration analysis module 613 can obtain an acceleration detrended fluctuation analysis value DFA and an acceleration sample entropy value SE1 as shown in FIG. 7.

In the present embodiment, the X-axis acceleration analysis module 611 can generate a first acceleration detrended fluctuation analysis value AX_DFA and a first acceleration sample entropy value AX_SE1. The Y-axis acceleration analysis module 612 can generate a second acceleration detrended fluctuation analysis value AY_DFA and a second acceleration sample entropy value AY_SE2. The Z-axis acceleration analysis module 613 can generate a third acceleration detrended fluctuation analysis value AZ_DFA and a third acceleration sample entropy value AZ_SE3. In other words, the second characteristic values of the present embodiment include the first acceleration detrended fluctuation analysis value AX_DFA, the first acceleration sample entropy value AX_SE1, the second acceleration detrended fluctuation analysis value AY_DFA, the second acceleration sample entropy value AY_SE2, the third acceleration detrended fluctuation analysis value AZ_DFA and the third acceleration sample entropy value AZ_SE3.

Nevertheless, enough teaching, suggestion, and implementation illustration regarding details of the related calculations for the acceleration detrended fluctuation analysis values and the acceleration sample entropy values of the present embodiment may be obtained with reference to common knowledge in the related art, which is not repeated hereinafter.

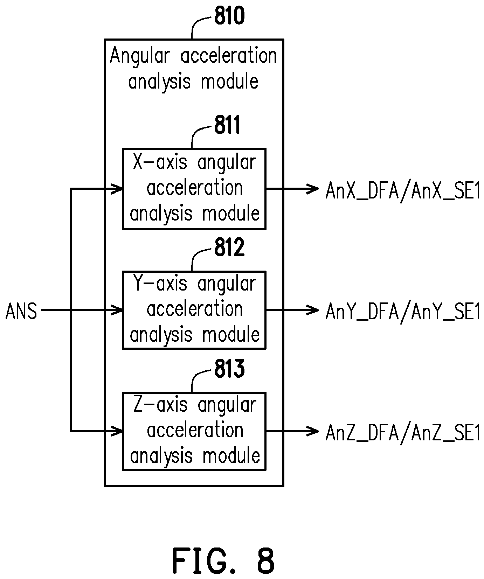

FIG. 8 illustrates a block diagram for analyzing an angular acceleration signal in an embodiment of the disclosure. With reference to FIG. 1 and FIG. 8, the storage device 120 can store, for example, an angular acceleration analysis module 810. In the present embodiment, the angular acceleration analysis module 810 includes an X-axis angular acceleration analysis module 811, a Y-axis angular acceleration analysis module 812 and a Z-axis angular acceleration analysis module 813. The angular acceleration sensor 150 can sense an angular acceleration signal AS of the wearer, wherein the angular acceleration sensor 150 may be, for example, a gyroscope. The angular acceleration analysis module 810 can analyze the angular acceleration signal AS to obtain respective angular acceleration values of X-axis (a first direction axis), Y-axis (a second direction axis) and Z-axis (a third direction axis), and obtain respective angular acceleration detrended fluctuation analysis values and respective angular acceleration sample entropy values by the X-axis angular acceleration analysis module 811, the Y-axis angular acceleration analysis module 812 and the Z-axis angular acceleration analysis module 813.

In the present embodiment, the X-axis angular acceleration analysis module 811 can generate a first angular acceleration detrended fluctuation analysis value AnX_DFA and a first angular acceleration sample entropy value AnX_SE1. The Y-axis angular acceleration analysis module 812 can generate a second angular acceleration detrended fluctuation analysis value AnY_DFA and a second angular acceleration sample entropy value AnY_SE2. The Z-axis angular acceleration analysis module 813 can generate a third angular acceleration detrended fluctuation analysis value AnZ_DFA and a third angular acceleration sample entropy value AnZ_SE3. In other words, the third characteristic values of the present embodiment include the first angular acceleration detrended fluctuation analysis value AnX_DFA, the first angular acceleration sample entropy value AnX_SE1, the second angular acceleration detrended fluctuation analysis value AnY_DFA, the second angular acceleration sample entropy value AnY_SE2, the third angular acceleration detrended fluctuation analysis value AnZ_DFA and the third angular acceleration sample entropy value AnZ_SE3.

Nevertheless, enough teaching, suggestion, and implementation illustration regarding details of the related calculations for the angular acceleration detrended fluctuation analysis values and the angular acceleration sample entropy values of the present embodiment may be obtained with reference to common knowledge in the related art, which is not repeated hereinafter.

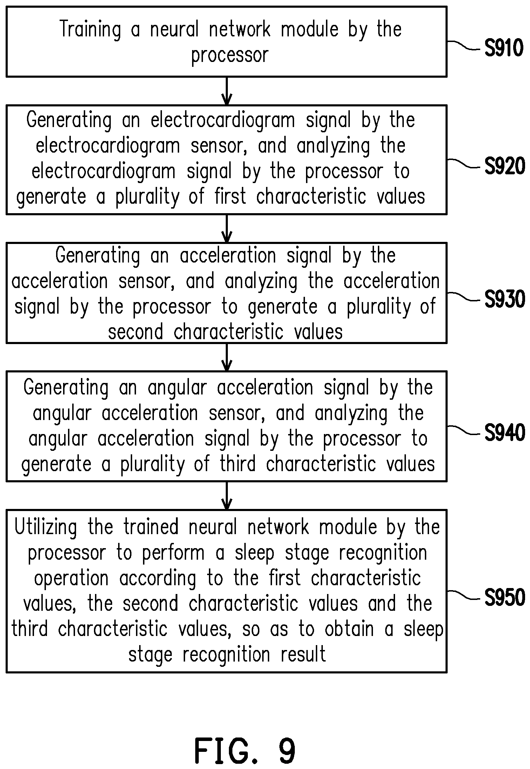

FIG. 9 illustrates a flowchart of a recognition method of sleep stage in an embodiment of the disclosure. With reference to FIG. 1 and FIG. 9, the recognition method of the present embodiment is at least adapted to the wearable device 100 of FIG. 1. In step S910, the processor 110 trains the neural network module 121. In step S920, the electrocardiogram sensor 130 generates an electrocardiogram signal, and the processor 110 analyzes the electrocardiogram signal to generate a plurality of first characteristic values. In step S930, the acceleration sensor 140 generates an acceleration signal, and the processor 110 analyzes the acceleration signal to generate a plurality of second characteristic values. In step S940, the angular acceleration sensor 150 generates an angular acceleration signal, and the processor 110 analyzes the angular acceleration signal to generate a plurality of third characteristic values. In step S950, the processor 110 utilizes the trained neural network module 121 to perform a sleep stage recognition operation according to the first characteristic values, the second characteristic values and the third characteristic values, so as to obtain a sleep stage recognition result.

In this way, the recognition method of the present embodiment can recognize the sleep stage of the wearer according to said 21 characteristic values sensed by the electrocardiogram sensor 130, the acceleration sensor 140 and the angular acceleration sensor 150. In the present embodiment, the sleep stage recognition result is one of the wakefulness stage, the first non-rapid eye movement stage, the second non-rapid eye movement stage, the third non-rapid eye movement stage and the rapid eye movement stage. Also, the wakefulness stage, the first non-rapid eye movement stage, the second non-rapid eye movement stage, the third non-rapid eye movement stage and the rapid eye movement stage are established by a polysomnography standard.

In addition, sufficient teaching, suggestion, and implementation regarding detailed features of each module and each sensor in the wearable device 100 of the present embodiment the disclosure may be obtained from the foregoing embodiments of FIG. 1 to FIG. 8, and thus related descriptions thereof are not repeated hereinafter.

In summary, the wearable device capable of recognizing sleep stage and the recognition method of sleep stage according to the disclosure can provide an accurate sleep recognition function. The wearable device includes the electrocardiogram sensor, the acceleration sensor and the angular acceleration sensor so the wearable device can sense the electrocardiogram information and posture variation of the wearer. The wearable device of the disclosure can use the trained neural network module to perform the sleep stage recognition operation according to the characteristic values provided by the electrocardiogram sensor, the acceleration sensor and the angular acceleration sensor. Moreover, the wearable device of the disclosure can integrate each of the sensors into one wearable item instead of complicated accessories. As a result, the wearable device of the disclosure is suitable for the wearer to conveniently and effectively monitor the sleep state in a home environment so as to obtain the recognition result of the sleep stage of the wearer.

## Claims

1. A wearable device (100) capable of recognizing sleep stage, comprising:
a processor (110), configured to train a neural network module (121);
an electrocardiogram sensor (130), coupled to the processor (110), and configured to generate an electrocardiogram signal (ECG), wherein the processor (110) is configured to analyze the electrocardiogram signal (ECG) to generate a plurality of first characteristic values;
an acceleration sensor (140), coupled to the processor (110), and configured to generate an acceleration signal (AS), wherein the processor (110) is configured to analyze the acceleration signal (AS) to generate a plurality of second characteristic values; and
an angular acceleration sensor (150), coupled to the processor (110), and configured to generate an angular acceleration signal (ANS), wherein the processor (110) is configured to analyze the angular acceleration signal (ANS) to generate a plurality of third characteristic values,
wherein the processor (110) is configured to utilize the trained neural network module (121) to perform a sleep stage recognition operation according to the first characteristic values, the second characteristic values and the third characteristic values, so as to obtain a sleep stage recognition result,
**characterised in that**
the processor (110) is configured to analyze the angular acceleration signal (ANS) by an X-axis angular acceleration analysis module (811), a Y-axis angular acceleration analysis module (812) and a Z-axis angular acceleration analysis module (813) to obtain a plurality of angular acceleration detrended fluctuation analysis values (AnX_DFA, AnY_DFA, AnZ_DFA) and a plurality of angular acceleration sample entropy values (AnX_SE1, AnY_SE1, AnZ_SE1) as the plurality of third characteristic values.

2. The wearable device (100) according to claim 1, wherein the processor (110) is configured to perform a heart rate variability analysis operation to analyze a heart beat interval variation (RRI) of the electrocardiogram signal (ECG), so as to obtain a low frequency signal (LF), a high frequency signal (HF), a detrended fluctuation analysis signal (DFA), a first sample entropy signal (SE1) and a second sample entropy signal (SE2),
wherein the first characteristic values are obtained from the low frequency signal (LF), the high frequency signal (HF), the detrended fluctuation analysis signal (DFA), the first sample entropy signal (SE1) and the second sample entropy signal (SE2).

3. The wearable device (100) according to claim 1 or 2, wherein the processor (110) is configured to perform a R-wave amplitude analysis operation to analyze a heart beat interval variation (RRI) of the electrocardiogram signal (ECG), so as to obtain a turning point ratio value (EDR_TPR) and a signal strength value (EDR_BR),
wherein the first characteristic values comprise the turning point ratio value (EDR_TPR) and the signal strength value (EDR_BR).

4. The wearable device (100) according to claim 3, wherein the processor (110) is configured to perform an adjacent R-waves difference analysis operation to analyze the heart beat interval variation (RRI) of the electrocardiogram signal (ECG), so as to obtain a mean value (EAD_mean) and a sample entropy value (EAD_SE1),
wherein the first characteristic values comprise the mean value (EAD_mean) and the sample entropy value (EAD_SE1).

5. The wearable device (100) according to any one of claims 1 to 4, wherein the processor (110) is configured to obtain a first acceleration value corresponding to a first direction axis, a second acceleration value corresponding to a second direction axis and a third acceleration value corresponding to a third direction axis according to the acceleration signal (AS),
wherein the processor (110) is configured to analyze the first acceleration value, the second acceleration value and the third acceleration value, so as to obtain a first acceleration detrended fluctuation analysis value (AX_DFA), a first acceleration sample entropy value (AX_SE1), a second acceleration detrended fluctuation analysis value (AY_DFA), a second acceleration sample entropy value (AY_SE1), a third acceleration detrended fluctuation analysis value (AZ_DFA) and a third acceleration sample entropy value (AZ_SE1),
wherein the second characteristic values comprise the first acceleration detrended fluctuation analysis value (AX_DFA), the first acceleration sample entropy value (AX_SE1), the second acceleration detrended fluctuation analysis value (AY_DFA), the second acceleration sample entropy value (AY_SE1), the third acceleration detrended fluctuation analysis value (AZ_DFA) and the third acceleration sample entropy value (AZ_SE1).

6. The wearable device (100) according to any one of claims 1 to 5, wherein the processor (110) is configured to obtain a first angular acceleration value corresponding to a first direction axis, a second angular acceleration value corresponding to a second direction axis and a third angular acceleration value corresponding to a third direction axis according to the angular acceleration signal (ANS),
wherein the processor (110) is configured to analyze the first angular acceleration value, the second angular acceleration value and the third angular acceleration value, so as to obtain a first angular acceleration detrended fluctuation analysis value (AnX_DFA), a first angular acceleration sample entropy value (AnX_SE1), a second angular acceleration detrended fluctuation analysis value (AnY_DFA), a second angular acceleration sample entropy value (AnY_SE1), a third angular acceleration detrended fluctuation analysis value (AnZ_DFA) and a third angular acceleration sample entropy value (AnZ_SE1),
wherein the third characteristic values comprise the first angular acceleration detrended fluctuation analysis value (AnX_DFA), the first angular acceleration sample entropy value (AnX_SE1), the second angular acceleration detrended fluctuation analysis value (AnY_DFA), the second angular acceleration sample entropy value (AnY_SE1), the third angular acceleration detrended fluctuation analysis value (AnZ_DFA) and the third angular acceleration sample entropy value (AnZ_SE1).

7. The wearable device (100) according to any one of claims 1 to 6, wherein the sleep stage recognition result is one of a wakefulness stage, a first non-rapid eye movement stage, a second non-rapid eye movement stage, a third non-rapid eye movement stage and a rapid eye movement stage, and the wakefulness stage, the first non-rapid eye movement stage, the second non-rapid eye movement stage, the third non-rapid eye movement stage and the rapid eye movement stage are established by a polysomnography standard.

8. The wearable device (100) according to any one of claims 1 to 7, wherein the processor (110) is configured to pre-train the neural network module (121) according to a plurality of sample data, and each of the sample data comprises another plurality of first characteristic values, another plurality of second characteristic values and another plurality of third characteristic values.

9. A recognition method of sleep stage, adapted to a wearable device (100), the wearable device (100) comprising a processor (110), an electrocardiogram sensor (130), an acceleration sensor (140) and an angular acceleration sensor (150), the method comprising:
training a neural network module (121) by the processor (110);
generating an electrocardiogram signal (ECG) by the electrocardiogram sensor (130), and analyzing the electrocardiogram signal (ECG) by the processor (110) to generate a plurality of first characteristic values;
generating an acceleration signal (AS) by the acceleration sensor (140), and analyzing the acceleration signal (AS) by the processor (110) to generate a plurality of second characteristic values;
generating an angular acceleration signal (ANS) by the angular acceleration sensor (150), and analyzing the angular acceleration signal (ANS) by the processor (110) to generate a plurality of third characteristic values; and
utilizing the trained neural network module (121) by the processor (110) to perform a sleep stage recognition operation according to the first characteristic values, the second characteristic values and the third characteristic values, so as to obtain a sleep stage recognition result,
**characterised in that**
the step of analyzing the angular acceleration signal (ANS) by the processor (110) to generate the third characteristic values comprises:
obtaining a plurality of angular acceleration detrended fluctuation analysis values (AnX_DFA, AnY_DFA, AnZ_DFA) and a plurality of angular acceleration sample entropy values (AnX_SE1, AnY_SE1, AnZ_SE1) as the plurality of third characteristic values by the processor (110) analyzing the angular acceleration signal (ANS) by an X-axis angular acceleration analysis module (811), a Y-axis angular acceleration analysis module (812) and a Z-axis angular acceleration analysis module (813).

10. The recognition method of sleep stage according to claim 9, wherein the step of analyzing the electrocardiogram signal (ECG) by the processor (110) to generate the first characteristic values comprises:
performing a heart rate variability analysis operation by the processor (110) to analyze a heart beat interval variation (RRI) of the electrocardiogram signal (ECG), so as to obtain a low frequency signal (LF), a high frequency signal (HF), a detrended fluctuation analysis signal (DFA), a first sample entropy signal (SE1) and a second sample entropy signal (SE2),
wherein the first characteristic values are obtained from the low frequency signal (LF), the high frequency signal (HF), the detrended fluctuation analysis signal (DFA), the first sample entropy signal (SE1) and the second sample entropy signal (SE2).

11. The recognition method of sleep stage according to claim 9 or 10, wherein the step of analyzing the electrocardiogram signal (ECG) by the processor (110) to generate the first characteristic values comprises:
performing a R-wave amplitude analysis operation by the processor (110) to analyze a heart beat interval variation (RRI) of the electrocardiogram signal (ECG), so as to obtain a turning point ratio value (EDR_TPR) and a signal strength value (EDR_BR),
wherein the first characteristic values comprise the turning point ratio value (EDR_TPR) and the signal strength value (EDR_BR).

12. The recognition method of sleep stage according to claim 11, further comprising:
performing an adjacent R-waves difference analysis operation by the processor (110) to analyze the heart beat interval variation (RRI) of the electrocardiogram signal (ECG), so as to obtain a mean value and a sample entropy value (EAD_mean),
wherein the first characteristic values comprise the mean value and the sample entropy value (EAD_SE1).

13. The recognition method of sleep stage according to any one of claims 9 to 12, wherein the step of analyzing the acceleration signal (AS) by the processor (110) to generate the second characteristic values comprises:
obtaining a first acceleration value corresponding to a first direction axis, a second acceleration value corresponding to a second direction axis and a third acceleration value corresponding to a third direction axis by the processor (110) according to the acceleration signal (AS); and
analyzing the first acceleration value, the second acceleration value and the third acceleration value by the processor (110), so as to obtain a first acceleration detrended fluctuation analysis value (AX_DFA), a first acceleration sample entropy value (AX_SE1), a second acceleration detrended fluctuation analysis value (AY_DFA), a second acceleration sample entropy value (AY_SE1), a third acceleration detrended fluctuation analysis value (AZ_DFA) and a third acceleration sample entropy value (AZ_SE1),
wherein the second characteristic values comprise the first acceleration detrended fluctuation analysis value (AX_DFA), the first acceleration sample entropy value (AX_SE1), the second acceleration detrended fluctuation analysis value (AY_DFA), the second acceleration sample entropy value (AY_SE1), the third acceleration detrended fluctuation analysis value (AZ_DFA) and the third acceleration sample entropy value (AZ_SE1).

14. The recognition method of sleep stage according to any one of claims 9 to 13, wherein the step of analyzing the angular acceleration signal (ANS) by the processor (110) to generate the third characteristic values comprises:
obtaining a first angular acceleration value corresponding to a first direction axis, a second angular acceleration value corresponding to a second direction axis and a third angular acceleration value corresponding to a third direction axis by the processor (110) according to the angular acceleration signal (ANS);
analyzing the first angular acceleration value, the second angular acceleration value and the third angular acceleration value by the processor (110), so as to obtain a first angular acceleration detrended fluctuation analysis value (AnX_DFA), a first angular acceleration sample entropy value (AnX_SE1), a second angular acceleration detrended fluctuation analysis value (AnY_DFA), a second angular acceleration sample entropy value (AnY_SE1), a third angular acceleration detrended fluctuation analysis value (AnZ_DFA) and a third angular acceleration sample entropy value (AnZ_SE1),
wherein the third characteristic values comprise the first angular acceleration detrended fluctuation analysis value (AnX_DFA), the first angular acceleration sample entropy value (AnX_SE1), the second angular acceleration detrended fluctuation analysis value (AnY_DFA), the second angular acceleration sample entropy value (AnY_SE1), the third angular acceleration detrended fluctuation analysis value (AnZ_DFA) and the third angular acceleration sample entropy value (AnZ_SE1).

15. The recognition method of sleep stage according to any one of claims 9 to 14, wherein the processor (110) pre-trains the neural network module (121) according to a plurality of sample data, and each of the sample data comprises another plurality of first characteristic values, another plurality of second characteristic values and another plurality of third characteristic values.

## Patentansprüche

1. Tragbare Vorrichtung (100), die fähig ist, ein Schlafstadium zu erkennen, welche Folgendes aufweist:
einen Prozessor (110), der konfiguriert ist, um ein neuronales Netzwerkmodul (121) zu trainieren;
einen Elektrokardiogrammsensor (130), der mit dem Prozessor (110) gekoppelt ist und konfiguriert ist, um ein Elektrokardiogrammsignal (ECG) zu erzeugen, wobei der Prozessor (110) konfiguriert ist, um das Elektrokardiogrammsignal (ECG) zu analysieren, um eine Vielzahl von ersten Charakteristikwerten zu erzeugen;
einen Beschleunigungssensor (140), der mit dem Prozessor (110) gekoppelt ist und konfiguriert ist, um ein Beschleunigungssignal (AS) zu erzeugen, wobei der Prozessor (110) konfiguriert ist, um das Beschleunigungsignal (AS) zu analysieren, um eine Vielzahl von zweiten Charakteristikwerten zu erzeugen; und
einen Winkelbeschleunigungssensor (150), der mit dem Prozessor (110) gekoppelt ist und konfiguriert ist, um ein Winkelbeschleunigungssignal (ANS) zu erzeugen, wobei der Prozessor (110) konfiguriert ist, um das Winkelbeschleunigungssignal (ANS) zu analysieren, um eine Vielzahl von dritten Charakteristikwerten zu erzeugen,
wobei der Prozessor (110) konfiguriert ist, um das trainierte neuronale Netzwerkmodul (121) zu verwenden, um einen Schlafstadiumserkennungsbetrieb gemäß den ersten Charakteristikwerten, den zweiten Charakteristikwerten und den dritten Charakteristikwerten auszuführen, um ein Schlafstadiumerkennungsergebnis zu erhalten,
**dadurch gekennzeichnet, dass**
der Prozessor (110) konfiguriert ist, um das Winkelbeschleunigungssignal (ANS) durch ein X-Achsen-Winkelbeschleunigungsanalysemodul (811), ein Y-Achsen-Winkelbeschleunigungsanalysemodul (812) und ein Z-Achsen-Winkelbeschleunigungsanalysemodul (813) zu analysieren, um eine Vielzahl von bezüglich der Winkelbeschleunigung vom Trend befreiten bzw. trendbereinigten Fluktuationsanalysewerten (AnX_DFA, AnY_DFA, AnZ_DFA) und eine Vielzahl von Winkelbeschleunigungssample-bzw. Winkelbeschleunigungsaufnahmeentropiewerten (AnX_SE1, AnY_SE1, AnZ_SE1) als die Vielzahl von dritten Charakteristikwerten zu erhalten.

2. Tragbare Vorrichtung (100) nach Anspruch 1, wobei der Prozessor (110) konfiguriert ist, um einen Herzschlagvariabilitätsanalysebetrieb auszuführen, um eine Herzschlagintervallvariation (RRI) des Elektrokardiogrammsignals (ECG) zu analysieren, um ein Niederfrequenzsignal (LF), ein Hochfrequenzsignal (HF), ein von einem Trend befreites bzw. trendbereinigtes Fluktuationsanalysesignal (DFA), ein erstes Sample- bzw. Aufnahmeentropiesignal (SE1) und ein zweites Aufnahmeentropiesignal (SE2) zu erhalten,
wobei die ersten Charakteristikwerte aus dem Niederfrequenzsignal (LF), dem Hochfrequenzsignal (HF), dem trendbereinigten Fluktuationsanalysesignal (DFA), dem ersten Sample- bzw. Aufnahmeentropiesignal (SE1) und dem zweiten Aufnahmeentropiesignal (SE2) erhalten werden.

3. Tragbare Vorrichtung (100) nach Anspruch 1 oder 2, wobei der Prozessor (110) konfiguriert ist, um einen R-Wellenamplitudenanalysebetrieb auszuführen, um eine Herzschlagintervallvariation (RRI) des Elektrokardiogrammsignals (ECG) zu analysieren, um einen Wendepunktverhältniswert (EDR_TPR) und einen Signalstärkewert (EDR_BR) zu erhalten,
wobei die ersten Charakteristikwerte den Wendepunktverhältniswert (EDR_TPR) und den Signalstärkewert (EDR_BR) aufweisen.

4. Tragbare Vorrichtung (100) nach Anspruch 3, wobei der Prozessor (110) konfiguriert ist, um einen Differenzanalysebetrieb für benachbarte R-Wellen auszuführen, um die Herzschlagintervallvariation (RRI) des Elektrokardiogrammsignals (ECG) zu analysieren, um einen Mittelwert (EAD_Mean) und einen Aufnahmeentropiewert (EAD_SE1) zu erhalten, wobei die ersten Charakteristikwerte den Mittelwert (EAD_Mean) und den Aufnahmeentropiewert (EAD_SE1) aufweisen.

5. Tragbare Vorrichtung (100) nach einem der Ansprüche 1 bis 4, wobei der Prozessor (110) konfiguriert ist, um einen ersten Beschleunigungswert entsprechend einer ersten Richtungsachse, einen zweiten Beschleunigungswert entsprechend einer zweiten Richtungsachse und einen dritten Beschleunigungswert entsprechend einer dritten Richtungsachse gemäß dem Beschleunigungssignal (AS) zu erhalten,
wobei der Prozessor (110) konfiguriert ist, um den ersten Beschleunigungswert, den zweiten Beschleunigungswert und den dritten Beschleunigungswert zu analysieren, um einen bezüglich der ersten Beschleunigung trendbereinigten Fluktuationsanalysewert (AX_DFA), einen ersten Beschleunigungsaufnameentropiewert (AX_SE1), einen bezüglich der zweiten Beschleunigung trendbereinigten Fluktuationsanalysewert (AY_DFA), einen zweiten Beschleunigungsaufnahmeentropiewert (AY_SE1), einen bezüglich der dritten Beschleunigung trendbereinigten Fluktuationsanalysewert (AZ_DFA) und einen dritten Beschleunigungsaufnahmeentropiewert (AZ_SE1) zu erhalten,
wobei die zweiten Charakteristikwerte den bezüglich der ersten Beschleunigung trendbereinigten Fluktuationsanalysewert (AX_DFA), den ersten Beschleunigungsaufnameentropiewert (AX_SE1), den bezüglich der zweiten Beschleunigung trendbereinigten Fluktuationsanalysewert (AY_DFA), den zweiten Beschleunigungsaufnahmeentropiewert (AY_SE1), den bezüglich der dritten Beschleunigung trendbereinigten Fluktuationsanalysewert (AZ_DFA) und den dritten Beschleunigungsaufnahmeentropiewert (AZ_SE1) aufweisen.

6. Tragbare Vorrichtung (100) nach einem der Ansprüche 1 bis 5, wobei der Prozessor (110) konfiguriert ist, um einen ersten Winkelbeschleunigungswert entsprechend einer ersten Richtungsachse, einen zweiten Winkelbeschleunigungswert entsprechend einer zweiten Richtungsachse und einen dritten Winkelbeschleunigungswert entsprechend einer dritten Richtungsachse gemäß dem Winkelbeschleunigungssignal (ANS) zu erhalten, wobei der Prozessor (110) konfiguriert ist, um den ersten Winkelbeschleunigungswert, den zweiten Winkelbeschleunigungswert und den dritten Winkelbeschleunigungswert zu analysieren, um einen bezüglich der ersten Winkelbeschleunigung trendbereinigten Fluktuationsanalysewert (AnX_DFA), einen ersten Winkelbeschleunigungsaufnameentropiewert (AnX_SE1), einen bezüglich der zweiten Winkelbeschleunigung trendbereinigten Fluktuationsanalysewert (AnY_DFA), einen zweiten Beschleunigungsaufnahmeentropiewert (AnY_SE1), einen bezüglich der dritten Winkelbeschleunigung trendbereinigten Fluktuationsanalysewert (AnZ_DFA) und einen dritten Beschleunigungsaufnahmeentropiewert (AnZ_SE1) zu erhalten,
wobei die dritten Charakteristikwerte den bezüglich der ersten Winkelbeschleunigung trendbereinigten Fluktuationsanalysewert (AnX_DFA), den ersten Winkelbeschleunigungsaufnameentropiewert (AnX_SE1), den bezüglich der zweiten Winkelbeschleunigung trendbereinigten Fluktuationsanalysewert (AnY_DFA), den zweiten Winkelbeschleunigungsaufnahmeentropiewert (AnY_SE1), den bezüglich der dritten Winkelbeschleunigung trendbereinigten Fluktuationsanalysewert (AnZ_DFA) und den dritten Winkelbeschleunigungsaufnahmeentropiewert (AnZ_SE1) aufweisen.

7. Tragbare Vorrichtung (100) nach einem der Ansprüche 1 bis 6, wobei das Schlafstadienerkennungsergebnis eines von Folgenden ist: ein Wachstadium, ein erstes Nicht-REM-Stadium, ein zweites Nicht-REM-Stadium, ein drittes Nicht-REM-Stadium und ein REM-Stadium (REM = Rapid Eye Movement = Schnelle Augenbewegung), und wobei das Wachstadium, das erste Nicht-REM-Stadium, das zweite Nicht-REM-Stadium, das dritte Nicht-REM-Stadium und das REM-Stadium durch einen Polysomnographiestandard eingerichtet bzw. festgestellt werden.

8. Tragbare Vorrichtung (100) nach einem der Ansprüche 1 bis 7, wobei der Prozessor (110) konfiguriert ist, um das neuronale Netzwerkmodul (121) gemäß einer Vielzahl von Sample- bzw. Aufnahmedaten vorzutrainieren, und wobei alle Aufnahmedaten eine andere Vielzahl von ersten Charakteristikwerten, eine andere Vielzahl von zweiten Charakteristikwerten und eine andere Vielzahl von dritten Charakteristikwerten aufweisen.

9. Schalfstadienerkennungsverfahren, welches an eine tragbare Vorrichtung (100) angepasst ist, wobei die tragbare Vorrichtung (100) einen Prozessor (110), einen Elektrokardiogrammsensor (130), einen Beschleunigungssensor (140) und einen Winkelbeschleunigungssensor (150) aufweist, wobei das Verfahren Folgendes aufweist:
Trainieren eines neuronalen Netzwerkmoduls (121) durch den Prozessor (110);
Erzeugen eines Elektrokardiogrammsignals (ECG) durch den Elektrokardiogrammsensor (130) und Analysieren des Elektrokardiogrammsignals (ECG) durch den Prozessor (110) zur Erzeugung einer Vielzahl von ersten Charakeristikwerten;
Erzeugen eines Beschleunigungssignals (AS) durch den Beschleunigungssensor (140) und Analysieren des Beschleunigungssignals (AS) durch den Prozessor (110) zur Erzeugung einer Vielzahl von zweiten Charakteristikwerten;
Erzeugen eines Winkelbeschleunigungsignals (ANS) durch den Winkelbeschleunigungssensor (150) und Analysieren des Winkelbeschleunigungssignals (ANS) durch den Prozessor (110) zur Erzeugung einer Vielzahl von dritten Charakteristikwerten; und
Verwenden des trainierten neuronalen Netzwerkmoduls (121) durch den Prozessor (110) zur Ausführung eines Schlafstadienerkennungsbetriebs gemäß den ersten Charakteristikwerten, den zweiten Charakteristikwerten und den dritten Charakteristikwerten, um ein Schlafstadienerkennungsergebnis zu erhalten,
**dadurch gekennzeichnet, dass**
der Schritt des Analysierens des Winkelbeschleunigungssignals (ANS) durch den Prozessor (110) zur Erzeugung der dritten Charakteristikwerte Folgendes aufweist:
Erhalten einer Vielzahl von bezüglich einer Winkelbeschleunigung vom Trend befreiten bzw. trendbereinigten Fluktuationsanalysewerten (AnX_DFA, AnY_DFA, AnZ_DFA) und einer Vielzahl von Winkelbeschleunigungssample-bzw. Winkelbeschleunigungsaufnahmeentropiewerten (AnX_SE1, AnY_SE1, AnZ_SE1) als die Vielzahl von dritten Charakteristikwerten durch den Prozessor (110), der das Winkelbeschleunigungssignal (ANS) durch ein X-Achsen-Winkelbeschleunigungsanalysemodul (811), ein Y-Achsen-Winkelbeschleunigungsanalysemodul (812) und ein Z-Achsen-Winkelbeschleunigungsanalysemodul (813) analysiert.

10. Schlafstadienerkennungsverfahren nach Anspruch 9, wobei der Schritt des Analysierens des Elektrokardiogrammsignals (ECG) durch den Prozessor (110) zur Erzeugung der ersten Charakteristikwerte Folgendes aufweist:
Ausführen eines Herzschlagvariabiltätsanalysebetriebs durch den Prozessor (110) zur Analyse einer Herzschlagintervallvariation (RRI) des Elektrokardiogrammsignals (ECG), um ein Niederfrequenzsignal (LF), ein Hochfrequenzsignal (HF), ein trendbereinigtes Fluktuationsanalysesignal (DFA), ein erstes Sample- bzw. Aufnahmeentropiesignal (SE1) und ein zweites Aufnahmeentropiesignal (SE2) zu erhalten,
wobei die ersten Charakteristikwerte aus dem Niederfrequenzsignal (LF), dem Hochfrequenzsignal (HF), dem trendbereinigten Fluktuationsanalysesignal (DFA), dem ersten Aufnahmeentropiesignal (SE1) und dem zweiten Aufnahmeentropiesignal (SE2) erhalten werden.

11. Schlafstadienerkennungsverfahren nach Anspruch 9 oder 10, wobei der Schritt des Analysierens des Elektrokardiogrammsignals (ECG) durch den Prozessor (110) zur Erzeugung der ersten Charakteristikwerte Folgendes aufweist:
Ausführen eines R-Wellenamplitudenanalysebetriebs durch den Prozessor (110), zum Analysieren einer Herzschlagintervallvariation (RRI) des Elektrokardiogrammsignals (ECG), um einen Wendepunktverhältniswert (EDR_TPR) und einen Signalstärkewert (EDR_BR) zu erhalten,
wobei die ersten Charakteristikwerte den Wendepunktverhältniswert (EDR_TPR) und den Signalstärkewert (EDR_BR) aufweisen.

12. Schlafstadienerkennungsverfahren nach Anspruch 11, welches weiter Folgendes aufweist:
Ausführen eines Differenzanalysebetriebs benachbarter R-Wellen durch den Prozessor (110) zum Analysieren der Herzschlagintervallvariation (RRI) des Elektrokardiogrammsignals (ECG), um einen Mittelwert (EAD_Mean) und einen Aufnahmeentropiewert zu erhalten,
wobei die ersten Charakteristikwerte den Mittelwert und den Aufnahmeentropiewert (EAD_SE1) aufweisen.

13. Schlafstadienerkennungsverfahren nach einem der Ansprüche 9 bis 12, wobei der Schritt des Analysierens des Beschleunigungssignals (AS) durch den Prozessor (110) zur Erzeugung der zweiten Charakteristikwerte Folgendes aufweisen:
Erlangen eines ersten Beschleunigungswertes entsprechend einer ersten Richtungsachse, eines zweiten Beschleunigungswertes entsprechend einer zweiten Richtungsachse und eines dritten Beschleunigungswertes entsprechend einer dritten Richtungsachse durch den Prozessor (110) gemäß dem Beschleunigungssignal (AS); und
Analysieren des ersten Beschleunigungswertes, des zweiten Beschleunigungswertes und des dritten Beschleunigungswertes durch den Prozessor (110), um einen bezüglich der ersten Beschleunigung von einem Trend befreiten bzw. trendbereinigten Fluktuationsanalysewert (AX_DFA), einen ersten Beschleunigungsaufnameentropiewert (AX_SE1), einen bezüglich der zweiten Beschleunigung trendbereinigten Fluktuationsanalysewert (AY_DFA), einen zweiten Beschleunigungsaufnahmeentropiewert (AY_SE1), einen bezüglich der dritten Beschleunigung trendbereinigten Fluktuationsanalysewert (AZ_DFA) und einen dritten Beschleunigungsaufnahmeentropiewert (AZ_SE1) zu erhalten,
wobei die zweiten Charakteristikwerte den bezüglich der ersten Beschleunigung trendbereinigten Fluktuationsanalysewert (AX_DFA), den ersten Beschleunigungsaufnameentropiewert (AX_SE1), den bezüglich der zweiten Beschleunigung trendbereinigten Fluktuationsanalysewert (AY_DFA), den zweiten Beschleunigungsaufnahmeentropiewert (AY_SE1), den bezüglich der dritten Beschleunigung trendbereinigten Fluktuationsanalysewert (AZ_DFA) und den dritten Beschleunigungsaufnahmeentropiewert (AZ_SE1) aufweisen.

14. Schlafstadienerkennungsverfahren nach einem der Ansprüche 9 bis 13, wobei der Schritt des Analysierens des Winkelbeschleunigungssignals (ANS) durch den Prozessor (110) zur Erzeugung der dritten Charakteristikwerte Folgendes aufweist:
Erlangen eines ersten Winkelbeschleunigungswertes entsprechend einer ersten Richtungsachse, eines zweiten Winkelbeschleunigungswertes entsprechend einer zweiten Richtungsachse und eines dritten Winkelbeschleunigungswertes entsprechend einer dritten Richtungsachse durch den Prozessor (110) gemäß dem Winkelbeschleunigungssignal (ANS);
Analysieren des ersten Winkelbeschleunigungswertes, des zweiten Winkelbeschleunigungswertes und des dritten Winkelbeschleunigungswertes durch den Prozessor (110), um einen bezüglich der ersten Winkelbeschleunigung von einem Trend befreiten bzw. trendbereinigten Fluktuationsanalysewert (AnX_DFA), einen ersten Winkelbeschleunigungsaufnameentropiewert (AnX_SE1), einen bezüglich der zweiten Winkelbeschleunigung trendbereinigten Fluktuationsanalysewert (AnY_DFA), einen zweiten Winkelbeschleunigungsaufnahmeentropiewert (AnY_SE1), einen bezüglich der dritten Winkelbeschleunigung trendbereinigten Fluktuationsanalysewert (AnZ_DFA) und einen dritten Winkelbeschleunigungsaufnahmeentropiewert (AnZ_SE1) zu erhalten,
wobei die dritten Charakteristikwerte den bezüglich der ersten Winkelbeschleunigung trendbereinigten Fluktuationsanalysewert (AnX_DFA), den ersten Winkelbeschleunigungsaufnameentropiewert (AnX_SE1), den bezüglich der zweiten Winkelbeschleunigung trendbereinigten Fluktuationsanalysewert (AnY_DFA), den zweiten Winkelbeschleunigungsaufnahmeentropiewert (AnY_SE1), den bezüglich der dritten Winkelbeschleunigung trendbereinigten Fluktuationsanalysewert (AnZ_DFA) und den dritten Winkelbeschleunigungsaufnahmeentropiewert (AnZ_SE1) aufweisen.

15. Schlafstadienerkennungsverfahren nach einem der Ansprüche 9 bis 14, wobei der Prozessor (110) das neuronale Netzwerkmodul (121) gemäß einer Vielzahl von Sample- bzw. Aufnahmedaten vortrainiert, und wobei alle Aufnahmedaten eine andere Vielzahl von ersten Charakteristikwerten, eine andere Vielzahl von zweiten Charakteristikwerten und eine andere Vielzahl von dritten Charakteristikwerten aufweisen.

## Revendications

1. Dispositif portable (100) capable de reconnaître une phase du sommeil, comprenant :
un processeur (110), configuré pour entraîner un module de réseau neuronal (121) ;
un capteur d'électrocardiogramme (130), couplé au processeur (110), et configuré pour générer un signal d'électrocardiogramme (ECG), dans lequel le processeur (110) est configuré pour analyser le signal d'électrocardiogramme (ECG) pour générer une pluralité de premières valeurs caractéristiques ;
un capteur d'accélération (140), couplé au processeur (110), et configuré pour générer un signal d'accélération (AS), dans lequel le processeur (110) est configuré pour analyser le signal d'accélération (AS) pour générer une pluralité de deuxièmes valeurs caractéristiques ; et
un capteur d'accélération angulaire (150), couplé au processeur (110), et configuré pour générer un signal d'accélération angulaire (ANS), dans lequel le processeur (110) est configuré pour analyser le signal d'accélération angulaire (ANS) pour générer une pluralité de troisièmes valeurs caractéristiques,
dans lequel le processeur (110) est configuré pour utiliser le module de réseau neuronal entraîné (121) pour mettre en œuvre une opération de reconnaissance de phase du sommeil selon les premières valeurs caractéristiques, les deuxièmes valeurs caractéristiques et les troisièmes valeurs caractéristiques, de sorte à obtenir un résultat de reconnaissance de phase du sommeil,
**caractérisé en ce que**
le processeur (110) est configuré pour analyser le signal d'accélération angulaire (ANS) par un module d'analyse d'accélération angulaire selon l'axe X (811), par un module d'analyse d'accélération angulaire selon l'axe Y (812) et par un module d'analyse d'accélération angulaire selon l'axe Z (813), pour obtenir une pluralité de valeurs d'analyse de fluctuations redressées d'accélération angulaire (AnX_DFA, AnY_DFA, AnZ_DFA) et une pluralité de valeurs d'entropie échantillon d'accélération angulaire (AnX_SE1, AnY_SE1, AnZ_SE1) en tant que pluralité de troisièmes valeurs caractéristiques.

2. Dispositif portable (100) selon la revendication 1, dans lequel le processeur (110) est configuré pour mettre en œuvre une opération d'analyse de variabilité de la fréquence cardiaque pour analyser une variation d'intervalle de battement cardiaque (RRI) du signal d'électrocardiogramme (ECG), de sorte à obtenir un signal à basse fréquence (LF), un signal à haute fréquence (HF), un signal d'analyse de fluctuations redressées (DFA), un premier signal d'entropie échantillon (SE1) et un deuxième signal d'entropie échantillon (SE2),
dans lequel les premières valeurs caractéristiques sont obtenues à partir du signal à basse fréquence (LF), du signal à haute fréquence (HF), du signal d'analyse de fluctuations redressées (DFA), du premier signal d'entropie échantillon (SE1) et du deuxième signal d'entropie échantillon (SE2).

3. Dispositif portable (100) selon la revendication 1 ou 2, dans lequel le processeur (110) est configuré pour mettre en œuvre une opération d'analyse d'amplitude d'onde R pour analyser une variation d'intervalle de battement cardiaque (RRI) du signal d'électrocardiogramme (ECG), de sorte à obtenir une valeur de rapport de point de retournement (EDR_TPR) et une valeur d'intensité de signal (EDR_BR),
dans lequel les premières valeurs caractéristiques comprennent la valeur de rapport de point de retournement (EDR_TPR) et la valeur d'intensité de signal (EDR_BR).

4. Dispositif portable (100) selon la revendication 3, dans lequel le processeur (110) est configuré pour mettre en œuvre une opération d'analyse de différence d'ondes R adjacentes pour analyser la variation d'intervalle de battement cardiaque (RRI) du signal d'électrocardiogramme (ECG), de sorte à obtenir une valeur moyenne (EAD_mean) et une valeur d'entropie échantillon (EAD_SE1),
dans lequel les premières valeurs caractéristiques comprennent la valeur moyenne (EAD_mean) et la valeur d'entropie échantillon (EAD_SE1).

5. Dispositif portable (100) selon l'une quelconque des revendications 1 à 4, dans lequel le processeur (110) est configuré pour obtenir une première valeur d'accélération correspondant à un premier axe de direction, une deuxième valeur d'accélération correspondant à un deuxième axe de direction et une troisième valeur d'accélération correspondant à un troisième axe de direction selon le signal d'accélération (AS),
dans lequel le processeur (110) est configuré pour analyser la première valeur d'accélération, la deuxième valeur d'accélération et la troisième valeur d'accélération, de sorte à obtenir une première valeur d'analyse de fluctuations redressées d'accélération (AX_DFA), une première valeur d'entropie échantillon d'accélération (AX_SE1), une deuxième valeur d'analyse de fluctuations redressées d'accélération (AY_DFA), une deuxième valeur d'entropie échantillon d'accélération (AY_SE1), une troisième valeur d'analyse de fluctuations redressées d'accélération (AZ_DFA) et une troisième valeur d'entropie échantillon d'accélération (AZ_SE1),
dans lequel les deuxièmes valeurs caractéristiques comprennent la première valeur d'analyse de fluctuations redressées d'accélération (AX_DFA), la première valeur d'entropie échantillon d'accélération (AX_SE1), la deuxième valeur d'analyse de fluctuations redressées d'accélération (AY_DFA), la deuxième valeur d'entropie échantillon d'accélération (AY_SE1), la troisième valeur d'analyse de fluctuations redressées d'accélération (AZ_DFA) et la troisième valeur d'entropie échantillon d'accélération (AZ_SE1).

6. Dispositif portable (100) selon l'une quelconque des revendications 1 à 5, dans lequel le processeur (110) est configuré pour obtenir une première valeur d'accélération angulaire correspondant à un premier axe de direction, une deuxième valeur d'accélération angulaire correspondant à un deuxième axe de direction et une troisième valeur d'accélération angulaire correspondant à un troisième axe de direction selon le signal d'accélération angulaire (ANS),
dans lequel le processeur (110) est configuré pour analyser la première valeur d'accélération angulaire, la deuxième valeur d'accélération angulaire et la troisième valeur d'accélération angulaire, de sorte à obtenir une première valeur d'analyse de fluctuations redressées d'accélération angulaire (AnX_DFA), une première valeur d'entropie échantillon d'accélération angulaire (AnX_SE1), une deuxième valeur d'analyse de fluctuations redressées d'accélération angulaire (AnY_DFA), une deuxième valeur d'entropie échantillon d'accélération angulaire (AnY_SE1), une troisième valeur d'analyse de fluctuations redressées d'accélération angulaire (AnZ_DFA) et une troisième valeur d'entropie échantillon d'accélération angulaire (AnZ_SE1),
dans lequel les troisièmes valeurs caractéristiques comprennent la première valeur d'analyse de fluctuations redressées d'accélération angulaire (AnX_DFA), la première valeur d'entropie échantillon d'accélération angulaire (AnX_SE1), la deuxième valeur d'analyse de fluctuations redressées d'accélération angulaire (AnY_DFA), la deuxième valeur d'entropie échantillon d'accélération angulaire (AnY_SE1), la troisième valeur d'analyse de fluctuations redressées d'accélération angulaire (AnZ_DFA) et la troisième valeur d'entropie échantillon d'accélération angulaire (AnZ_SE1).

7. Dispositif portable (100) selon l'une quelconque des revendications 1 à 6, dans lequel le résultat de la reconnaissance de la phase du sommeil est une phase parmi une phase d'éveil, une première phase de mouvements oculaires non rapides, une deuxième phase de mouvements oculaires non rapides, une troisième phase de mouvements oculaires non rapides et une phase de mouvements oculaires rapides, et la phase d'éveil, la première phase de mouvements oculaires non rapides, la deuxième phase de mouvements oculaires non rapides, la troisième phase de mouvements oculaires non rapides et la phase de mouvements oculaires rapides sont établies par une norme de polysomnographie.

8. Dispositif portable (100) selon l'une quelconque des revendications 1 à 7, dans lequel le processeur (110) est configuré pour pré-entraîner le module de réseau neuronal (121) selon une pluralité de données échantillon, et chacune des données échantillon comprend une autre pluralité de premières valeurs caractéristiques, une autre pluralité de deuxièmes valeurs caractéristiques et une autre pluralité de troisièmes valeurs caractéristiques.

9. Procédé de reconnaissance de phase du sommeil, adapté à un dispositif portable (100), le dispositif portable (100) comprenant un processeur (110), un capteur d'électrocardiogramme (130), un capteur d'accélération (140) et un capteur d'accélération angulaire (150), le procédé comprenant les étapes suivantes :
l'apprentissage d'un module de réseau neuronal (121) par le processeur (110) ;
la génération d'un signal d'électrocardiogramme (ECG) par le capteur d'électrocardiogramme (130), et l'analyse du signal d'électrocardiogramme (ECG) par le processeur (110) pour générer une pluralité de premières valeurs caractéristiques ;
la génération d'un signal d'accélération (AS) par le capteur d'accélération (140), et l'analyse du signal d'accélération (AS) par le processeur (110) pour générer une pluralité de deuxièmes valeurs caractéristiques ;
la génération d'un signal d'accélération angulaire (ANS) par le capteur d'accélération angulaire (150), et l'analyse du signal d'accélération angulaire (ANS) par le processeur (110) pour générer une pluralité de troisièmes valeurs caractéristiques ; et
l'utilisation du module de réseau neuronal entraîné (121) par le processeur (110) pour mettre en œuvre une opération de reconnaissance de phase du sommeil selon les premières valeurs caractéristiques, les deuxièmes valeurs caractéristiques et les troisièmes valeurs caractéristiques, de sorte à obtenir un résultat de reconnaissance de phase du sommeil,
**caractérisé en ce que**
l'étape d'analyse du signal d'accélération angulaire (ANS) par le processeur (110) pour générer les troisièmes valeurs caractéristiques comprend :
l'obtention d'une pluralité de valeurs d'analyse de fluctuations redressées d'accélération angulaire (AnX_DFA, AnY_DFA, AnZ_DFA) et d'une pluralité de valeurs d'entropie échantillon d'accélération angulaire (AnX_SE1, AnY_SE1, AnZ_SE1) en tant que pluralité de troisièmes valeurs caractéristiques par le processeur (110) en analysant le signal d'accélération angulaire (ANS) par un module d'analyse d'accélération angulaire selon un axe X (811), par un module d'analyse d'accélération angulaire selon un axe Y (812) et par un module d'analyse d'accélération angulaire selon un axe Z (813).

10. Procédé de reconnaissance de phase du sommeil selon la revendication 9, dans lequel l'étape d'analyse du d'électrocardiogramme (ECG) par le processeur (110) pour générer les premières valeurs caractéristiques comprend l'étape suivante :
la mise en œuvre d'une opération d'analyse de variabilité de la fréquence cardiaque par le processeur (110) pour analyser une variation d'intervalle de battement cardiaque (RRI) du signal d'électrocardiogramme (ECG), de sorte à obtenir un signal à basse fréquence (LF), un signal à haute fréquence (HF), un signal d'analyse de fluctuations redressées (DFA), un premier signal d'entropie échantillon (SE1) et un deuxième signal d'entropie échantillon (SE2),
dans lequel les premières valeurs caractéristiques sont obtenues à partir du signal à basse fréquence (LF), du signal à haute fréquence (HF), du signal d'analyse de fluctuations redressées (DFA), du premier signal d'entropie échantillon (SE1) et du deuxième signal d'entropie échantillon (SE2).

11. Procédé de reconnaissance de phase du sommeil selon la revendication 9 ou 10, dans lequel l'étape d'analyse du signal d'électrocardiogramme (ECG) par le processeur (110) pour générer les premières valeurs caractéristiques comprend l'étape suivante :
la mise en oeuvre d'une opération d'analyse d'amplitude d'onde R par le processeur (110) pour analyser une variation d'intervalle de battement cardiaque (RRI) du signal d'électrocardiogramme (ECG), de sorte à obtenir une valeur de rapport de point de retournement (EDR_TPR) et une valeur d'intensité de signal (EDR_BR),
dans lequel les premières valeurs caractéristiques comprennent la valeur de rapport de point de retournement (EDR_TPR) et la valeur d'intensité de signal (EDR_BR).

12. Procédé de reconnaissance de phase du sommeil selon la revendication 11, comprenant en outre l'étape suivante :
la mise en œuvre d'une opération d'analyse de différence d'ondes R adjacentes par le processeur (110) pour analyser la variation d'intervalle de battement cardiaque (RRI) du signal d'électrocardiogramme (ECG), de sorte à obtenir une valeur moyenne (EAD_mean) et une valeur d'entropie échantillon,
dans lequel les premières valeurs caractéristiques comprennent la valeur moyenne et la valeur d'entropie échantillon (EAD_SE1).

13. Procédé de reconnaissance de phase du sommeil selon l'une quelconque des revendications 9 à 12, dans lequel l'étape d'analyse du signal d'accélération (AS) par le processeur (110) pour générer les deuxièmes valeurs caractéristiques comprend les étapes suivantes :
l'obtention d'une première valeur d'accélération correspondant à un premier axe de direction, d'une deuxième valeur d'accélération correspondant à un deuxième axe de direction et d'une troisième valeur d'accélération correspondant à un troisième axe de direction par le processeur (110) selon le signal d'accélération (AS) ; et
l'analyse de la première valeur d'accélération, de la deuxième valeur d'accélération et de la troisième valeur d'accélération par le processeur (110), de sorte à obtenir une première valeur d'analyse de fluctuations redressées d'accélération (AX_DFA), une première valeur d'entropie échantillon d'accélération (AX_SE1), une deuxième valeur d'analyse de fluctuations redressées d'accélération (AY_DFA), une deuxième valeur d'entropie échantillon d'accélération (AY_SE1), une troisième valeur d'analyse de fluctuations redressées d'accélération (AZ_DFA) et une troisième valeur d'entropie échantillon d'accélération (AZ_SE1),
dans lequel les deuxièmes valeurs caractéristiques comprennent la première valeur d'analyse de fluctuations redressées d'accélération (AX_DFA),la première valeur d'entropie échantillon d'accélération (AX_SE1), la deuxième valeur d'analyse de fluctuations redressées d'accélération (AY_DFA), la deuxième valeur d'entropie échantillon d'accélération (AY_SE1), la troisième valeur d'analyse de fluctuations redressées d'accélération (AZ_DFA) et la troisième valeur d'entropie échantillon d'accélération (AZ_SE1).

14. Procédé de reconnaissance de phase du sommeil selon l'une quelconque des revendications 9 à 13, dans lequel l'étape d'analyse du signal d'accélération angulaire (ANS) par le processeur (110) pour générer les troisièmes valeurs caractéristiques comprend les étapes suivantes :
l'obtention d'une première valeur d'accélération angulaire correspondant à un premier axe de direction, d'une deuxième valeur d'accélération angulaire correspondant à un deuxième axe de direction et d'une troisième valeur d'accélération angulaire correspondant à un troisième axe de direction par le processeur (110) selon le signal d'accélération angulaire (ANS) ;
l'analyse de la première valeur d'accélération angulaire, de la deuxième valeur d'accélération angulaire et de la troisième valeur d'accélération angulaire par le processeur (110), de sorte à obtenir une première valeur d'analyse de fluctuations redressées d'accélération angulaire (AnX_DFA), une première valeur d'entropie échantillon d'accélération angulaire (AnX_SE1), une deuxième valeur d'analyse de fluctuations redressées d'accélération angulaire (AnY_DFA), une deuxième valeur d'entropie échantillon d'accélération angulaire (AnY_SE1), une troisième valeur d'analyse de fluctuations redressées d'accélération angulaire (AnZ_DFA) et une troisième valeur d'entropie échantillon d'accélération angulaire (AnZ_SE1),
dans lequel les troisièmes valeurs caractéristiques comprennent la première valeur d'analyse de fluctuations redressées d'accélération angulaire (AnX_DFA), la première valeur d'entropie échantillon d'accélération angulaire (AnX_SE1), la deuxième valeur d'analyse de fluctuations redressées d'accélération angulaire (AnY_DFA), la deuxième valeur d'entropie échantillon d'accélération angulaire (AnY_SE1), la troisième valeur d'analyse de fluctuations redressées d'accélération angulaire (AnZ_DFA) et la troisième valeur d'entropie échantillon d'accélération angulaire (AnZ_SE1).

15. Procédé de reconnaissance de phase du sommeil selon l'une quelconque des revendications 9 à 14, dans lequel le processeur (110) pré-entraîne le module de réseau neuronal (121) selon une pluralité de données échantillon, et chacune des données échantillon comprend une autre pluralité de premières valeurs caractéristiques, une autre pluralité de deuxièmes valeurs caractéristiques et une autre pluralité de troisièmes valeurs caractéristiques.
